# EUROPEAN PATENT SPECIFICATION

(11) **EP 1 309 302 B1**
(45) Date of publication and mention of the grant of the patent: **21.11.2007**
(21) Application number: 01944090.8
(22) Date of filing: 28.06.2001
(51) Int. Cl.: A61F 13/64

(54) **BELTED ABSORBENT ARTICLE**
ABSORBIERENDER ARTIKEL MIT GÜRTEL
ARTICLE ABSORBANT A CEINTURE

(30) Priority: 13.07.2000 SE 0002660
(43) Date of publication of application: 14.05.2003
(73) Proprietor: SCA Hygiene Products AB, 405 03 Göteborg (SE)
(72) Inventor: ALMBERG, Christian, S-435 38 Mölnlycke (SE); BÄCK, Lucas, S-414 57 Göteborg (SE)
(74) Representative: Hammond, Andrew David
(86) International application number: PCT/SE2001/001496
(87) International publication number: WO 2002/005739

(56) References cited:
- WO-A1-99/37263
- US-A- 5 904 673
- *dScientific and Technical Terms*d, 5th. ed (1994); McGraw-Hill, Inc.; p. A8

## Description

### TECHNICAL FIELD:

The present invention relates to a belted absorbent article according to the preamble of claim 1.

### BACKGROUND OF THE INVENTION:

Conventional diapers for young children generally comprise an absorbent structure made up of an absorbent core sandwiched between a liquid pervious topsheet and a liquid-impervious backsheet. The absorbent structure has a front panel generally covering the child's abdomen when worn and a rear panel generally covering the child's buttocks. The front and rear panels terminate in front and rear end regions respectively. In order to secure the diaper about the child, the front and rear end regions are provided with cooperating fastening means, for example in the form of a hook-and-loop fastening system or, alternatively, an adhesive tape tab system on the rear end region cooperating with a landing zone on the front end region. To fit the diaper to the child, the child is normally laid down on his/her back, the diaper is inserted under the child such that the rear panel covers the child's buttocks, the front panel is drawn between the child's legs to cover the abdomen and the fastening system is employed to join the longitudinal sides of the respective front and rear end regions together.

Since it is almost exclusively a guardian who changes a child's diaper, the above-described method of securing a diaper to a child is generally fully satisfactory. However, particularly for incontinent adults who are capable of changing their own diapers, such a method of securing an absorbent article about the body is inconvenient, primarily because the act of securing the absorbent article about the adult body is conducted when standing. Thus, it is awkward for the wearer to simultaneously hold the absorbent article to the body in its correct orientation whilst fastening the front and rear end regions to each other.

To at least partially alleviate the above-mentioned inconvenience, belted absorbent articles have been proposed. For example, a belted absorbent article is disclosed in FR-A-2 586 558 in which a one-piece belt is attached to the backsheet of an absorbent structure. The article is secured about the wearer by bringing the article to the rear of the wearer to thereby cover the buttocks, fastening the belt about the waist of the wearer and then drawing the front end region of the absorbent structure between the legs of the wearer and attaching the front end region to fastening means on the belt.

EP-B-0 729 329 discloses a belted absorbent article in which, rather than using a single belt attached to the absorbent structure, two belt halves are employed, with each belt half extending from a respective longitudinal edge of the absorbent structure. An advantage of this construction over that disclosed in FR-A-2 586 558 is that less belt material is needed. However, because two belt halves are employed, the joint between each belt half and the absorbent structure must be capable of withstanding the tension forces to which the belt halves are typically subjected during use.

Particularly in terms of adult incontinence diapers, the size of wearers varies greatly. In order to rationalize production and storage as much as possible, it is preferable to have as few product size variations as possible. This implies, on the other hand, that each product size must be capable of fitting wearers of widely diverging shape and size. As a consequence, the forces to which the belt halves are subjected during use will be, at least to a certain extent, dependent on the size of the wearer. To keep manufacturing costs low, it is of course advantageous to use as little material as possible. Nevertheless, a product must be sufficiently strong to fulfil its intended purpose.

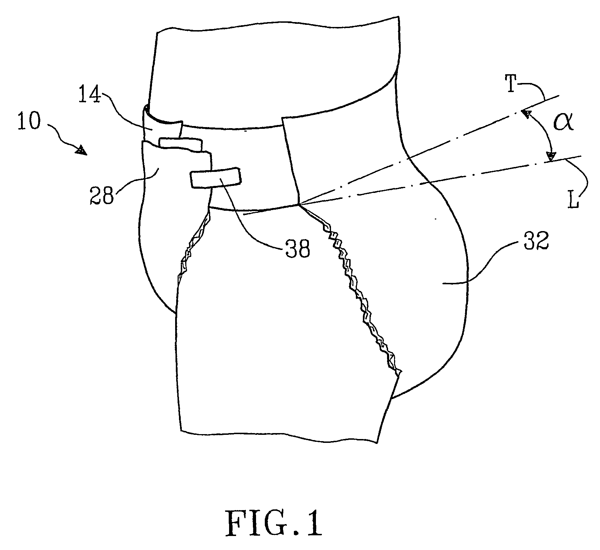

As is apparent from Fig. 1, when a belted absorbent article 10 is secured to a wearer, an angle α is created between, in effect, the longitudinal axis L of the belt half and the transverse axis T of the absorbent structure (it being assumed in the illustrated embodiment that the longitudinal edge of the belt half is parallel to the longitudinal axis of the belt half). Depending on the size of the wearer in relation to the size of the article, the value of α will vary. The greater the size of wearer, the greater the value of α will be. As the angle α increases, an increasing shear force is applied to the joint between the belt half and the absorbent structure.

The present applicant has discovered that, in order for a belted absorbent article to function satisfactorily, though whilst still keeping manufacturing costs as low as reasonably possible, the joint between each belt half and the absorbent structure must meet certain minimum requirements depending on the angle α.

### SUMMARY OF THE INVENTION:

It is therefore an object of the present invention to provide a belted absorbent article which functions satisfactorily though whilst still keeping manufacturing costs as low as reasonably possible.

This object is achieved by means of the belted absorbent article comprising an absorbent structure extending about a first longitudinal axis, the absorbent structure including a topsheet, a backsheet and an absorbent batt disposed between the topsheet and the backsheet, the absorbent structure having a transverse axis dividing the absorbent structure into a front panel terminating in a front end region and a rear panel terminating in a rear end region, the absorbent structure being delimited by opposed longitudinal edges and opposed transverse edges, and a pair of opposed belt halves attached to the absorbent structure at the rear end region of the rear panel by a respective joint, each belt half extending about a second longitudinal axis such that each belt half extends outwardly from a respective longitudinal edge of the absorbent structure. The joint between each belt half and the absorbent structure is designed such that when each belt half is subjected to a tension force of 35 N acting along the second longitudinal axis and the second longitudinal axis creates an angle α to said transverse axis of said absorbent structure, the following minimum average release times (t) of each said belt half from said absorbent structure are attained:
when α = 10°, minimum t = 720 seconds;
when α = 20°, minimum t = 330 seconds;
when α = 25°, minimum t = 240 seconds;
when α = 30°, minimum t = 180 seconds; and
when α = 40°, minimum t = 75 seconds.

In a preferred embodiment of the invention, the following minimum average release times (t) of each belt half from the absorbent structure are attained:
when α = 10°, minimum t = 740 seconds;
when α = 20°, minimum t = 340 seconds;
when α = 25°, minimum t = 245 seconds;
when α = 30°, minimum t = 190 seconds; and
when α = 40°, minimum t = 80 seconds.

Thus, in accordance with the invention, by ensuring that the joint between each belt half and the absorbent structure can withstand a force of 35 N at predetermined values of the angle α for a minimum average release time per chosen angle, it has been found in practice that failure of the belted absorbent article due to loosening of the belt halves does not occur, at least not during normal use.

Further preferred embodiments of the belted absorbent article according to the present invention are detailed in the remaining dependent claims.

### BRIEF DESCRIPTION OF THE DRAWINGS:

The invention will be described in the following in greater detail by way of example only and with reference to the attached drawings, in which:
- Fig. 1: is a schematic side view of a belted absorbent article secured to a wearer;
- Fig. 2: is a schematic plan view of a belted absorbent article according to the present invention;
- Fig. 3: is an enlarged view of the joint between a belt half and the absorbent structure of the belted absorbent article according to the present invention;
- Fig. 4: is a schematic view of a cut out section of the article according to the present invention being subjected to a test procedure;.
- Fig. 5: is a schematic plan view illustrating sections of the belted absorbent article according to the present invention which are to be cut out and tested;
- Fig. 6: is a schematic elevational view of a test rig for subjecting cut out sections of the belted absorbent product to a test procedure;
- Fig. 7: is an engineering drawing in the form of an elevational view of one component of the test rig of Fig. 6;
- Fig. 8: is an engineering drawing in the form of an end view of the component of Fig. 7;
- Fig. 9: is an enlarged view of a portion of the component of Fig. 8;
- Fig. 10: is an engineering drawing in the form of an elevational view of a second component of the test rig of Fig. 6;
- Fig. 11: is an engineering drawing in the form of an end view of the component of Fig. 10;
- Fig. 12: is an enlarged view of a portion of the component of Fig. 11;
- Fig. 13: is an engineering drawing in the form of an elevational view of a third component of the test rig of Fig. 6; and
- Fig. 14: is an engineering drawing in the form of an end view of the component of Fig. 13;

### DETAILED DESCRIPTION OF PREFERRED EMBODIMENTS:

In the drawings, reference numeral 10 generally denotes a belted absorbent article according to the present invention. The expression "belted" implies that the article is provided with means which pass around at least a portion of the waist of a wearer when worn and which means meet to thereby secure the article around the waist of the wearer. In the present invention, these means are belt halves 12, 14. Preferably, the invention is to be practised on a disposable such article. In this respect, the term "disposable" means any article which is not intended to be laundered and which is normally discarded once removed from the wearer.

In addition to the belt halves 12, 14, the belted absorbent article further comprises an absorbent structure 16 extending about a first longitudinal axis 18. Thus, when the article is being worn, the first longitudinal axis will generally bisect the wearer into a left hand side and a right hand side when viewed from above. The absorbent structure 16 includes a topsheet 20, a backsheet 22 and an absorbent batt 24 disposed between the topsheet and the backsheet. The specific components used to form the absorbent structure may be any of the types commonly used for such purposes. For example, a suitable topsheet 20 may be any soft flexible non-irritating liquid permeable material, such as woven or nonwoven webs of natural fibres (e.g. wood or cotton fibres), synthetic fibres (e.g. polyester or polypropylene fibres or coform fibres), combinations of natural and synthetic fibres, apertured films or porous foams. Preferably, the topsheet 20 is manufactured from a nonwoven spunbonded polypropylene material having a basis weight of less than 20 g/m². Nevertheless, when the topsheet comprises a nonwoven web, the nonwoven web may instead be carded, wet-laid, meltblown, hydroformed or hydroentangled. The backsheet 22 is impervious to liquids and is preferably manufactured from a thin plastic film such as polyethylene, polypropylene, polyvinylchloride, or the like, or composite materials such as a film coated nonwoven material. The absorbent core 24 may be made in widely varying sizes and shapes and may comprise any suitable wettable hydrophilic fibres such as cellulosic fibres, possibly blended with synthetic polyolefin fibres. In certain embodiments, the absorbent core 24 may comprise a mixture of superabsorbent hydrogel-forming particles mixed with the hydrophilic fibres.

The topsheet 20 and the backsheet 22 are connected together, either directly or indirectly, using any suitable known means. For example, the topsheet and the backsheet can be affixed directly to each other in selected areas using continuous or patterned layers of adhesive. The adhesive layer or layers may be sprayed or extruded in lines or dots.

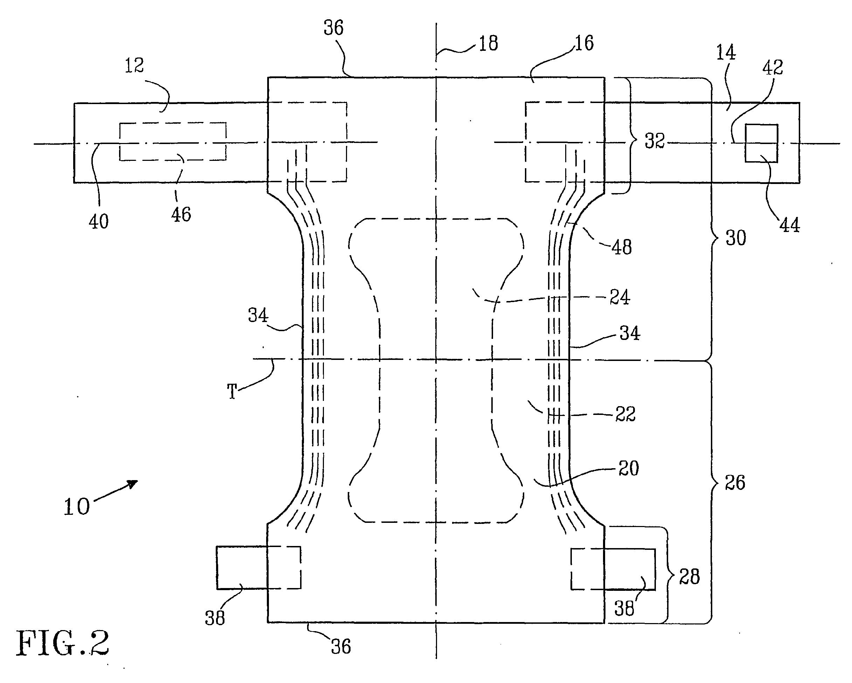

As is most clearly apparent from Fig. 2, the absorbent structure has a transverse axis T dividing the structure into a front panel 26 terminating in a front end region 28 and a rear panel 30 terminating in a rear end region 32. The absorbent structure is delimited by opposed longitudinal edges 34 and opposed transverse edges 36. Projecting from its opposed longitudinal edges 34, the front end region 28 displays fastening tabs 38. In a manner which will be explained in greater detail below, the fastening tabs 38 form part of a fastening system and are arranged to cooperate with corresponding parts of the fastening system on the belt halves 12, 14. Although the fastening tabs 38 are shown projecting from the longitudinal edges 34 of the front panel of the absorbent structure 16, it is to be understood that the tabs may instead project from the transverse edge 36. In a further alternative embodiment, the fastening tabs 38 may be in the form of one or more patches affixed to the topsheet 20 on the front panel 26.

Each belt half extends about a respective longitudinal axis (hereinafter referred to as the second longitudinal axis) 40, 42, with the belt halves being joined to the absorbent structure 16 such that the two belt halves 12, 14 extend generally perpendicularly with respect to the first longitudinal axis 18 of the absorbent structure 16. Accordingly, in the shown embodiment, the two belt halves 12, 14 extend generally perpendicularly from the longitudinal edges 34 at the rear end region 32 of the rear panel 30 of the absorbent structure 16. It is, however, to be understood that the belt halves may be manufactured such that they form an angle to either the first longitudinal axis 18 or to the longitudinal edges 34. The belt halves may be made of any suitable material or material combinations. In an exemplary embodiment, the belt halves are made from a laminate of an embossed transparent polypropylene film and a thermobonded nonwoven web of 30 g/m² basis weight. For improved wearer comfort, the belt halves are attached to the absorbent structure such that, when worn, the nonwoven web faces the wearer. In another embodiment, the belt halves may be made exclusively of nonwoven material. Depending on the intended size of the wearer, each belt half 12, 14 has a transverse extension of typically 10 to 12 cm and a longitudinal extension (i.e. the extension beyond the longitudinal edge 34 of the absorbent structure) of, for example, 35 cm. The belt halves are preferably identical, though it is to be understood that the absorbent article of the present invention may also have belt halves or different length and/or width.

The inside surface, i.e. that surface of the belt half facing the wearer when worn, of one belt half (belt half 14 in Fig. 2) is provided with a first fastening means 44 for releasable engagement with a complementary second fastening means 46 on the outer surface of the other belt half 12 to thereby allow the belt to be secured around the waist of the wearer. The actual type of belt fastening means may be any of those known in the art, for example a hook-and loop system, an adhesive system, a system of buttons and button-holes, etc. Irrespective of the type of fastening system employed, the fastening system should be capable of allowing adjustment of the tension of the belt halves around the waist of the wearer. Thus, in the embodiment shown on Fig. 2, the first fastening means is a patch of hook material while the second fastening means is a strip of loop material extending over a significant length of the belt half 12. It will of course be apparent to the skilled person that more than one patch of hook material may be used and that the strip of loop material may be a plurality of smaller patches of loop material.

The surface of the belt halves remote from the wearer when worn, i.e. the outer surface of the belt halves, is provided with means (not shown) forming a part of the fastening system of the absorbent article. The means on the belt halves is complementary to, and therefore dependent on, the type of fastening tab 38 employed in the front end region 28 of the front panel 26. Thus, if the fastening tabs 38 are adhesive tabs, at least a region of the outer surface of the belt halves 12, 14 must be capable of releasable engagement of the adhesive tabs. In the case in which the outer surface of the belt halves is a plastics film, it is advantageous to provide a reinforced so-called landing zone or zones for the tabs to engage with. If the fastening tabs 38 are hook tabs of a hook-and-loop fastening system, the outer surface of the belt halves will be provided with, or made from, a loop material for engagement with the hook fastening tabs. Another possibility is that the outer surface of the belt halves is provided with patches of hook material for engagement with loop material of the topsheet 20 or patches of loop material attached to the topsheet in the.front end region 28 of the front panel 26.

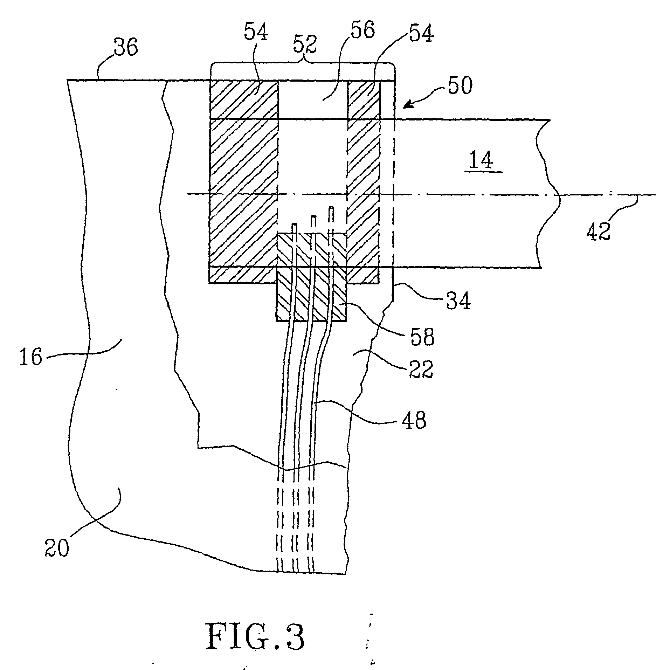

In a manner known per se in the art, the absorbent article of the present invention may be provided with elasticated leg cuffs to thereby provide improved sealing of the article around the legs of the wearer when worn. As is most clearly shown in Figs. 2 and 3, the absorbent structure 16 is provided with elastic ribbons 48 or strands affixed between the topsheet 20 and the backsheet 22. The elastic ribbons extend from an area in the rear end region 32 remote from the transverse edge 36 along a path generally parallel to the longitudinal edges 34 of the absorbent structure to an area in the front end region 28 remote from its transverse edge 36. In the illustrated embodiment, three elastic ribbons 48 or strands are shown for each leg cuff. The person skilled in the art will, however, appreciate that the actual number of elastic ribbons may be varied, as may their size. Thus, although the ribbons are illustrated as threads, it will be appreciated that strips of elastic material may be used instead.

Each belt half 12, 14 is attached to the absorbent structure 16 at the rear end region 32 of the rear panel 30 by a respective joint, generally denoted by reference numeral 50. One example of a joint 50 which may be used in the absorbent article of the present invention is illustrated in Fig. 3. It is to be understood that, although the joint will be described below only with reference to one belt half 14, an identical joint may be used for the other belt half 12. A length 52 of the belt half 14 is attached to the absorbent structure 16 at the rear end region 32 between the topsheet 20 and the backsheet 22. The length 52 of the belt half thus sandwiched between the topsheet and backsheet will vary depending on the size of the absorbent article, but is typically between 8 and 10 cm. When, and in what order, the belt half is attached to the topsheet and backsheet will depend on the chosen manufacturing process. The belt half 14 is generally spaced a short distance, for example about 1 cm, from the transverse edge 36 of the absorbent structure and, prior to being worn, the longitudinal axis 42 of the belt half may extend substantially parallel to the transverse edge. Thus, in the illustrated embodiment, the longitudinal axis of the belt half extends parallel to the transverse axis T of the absorbent structure. The joint 50 between the belt half 14 and the rear end region 32 comprises two spaced substantially parallel lines of attachment 54 between the belt half and the backsheet 22. The line of attachment 54 adjacent the longitudinal edge 34 of the absorbent structure 16 may be spaced a short distance from the longitudinal edge. This line of attachment is generally narrower than the line of attachment remote from the longitudinal edge 34 of the absorbent structure. The two spaced lines of attachment 54 generally extend from the transverse edge 36 of the rear end region parallel to the longitudinal axis 18 of the absorbent structure across the entire width of the belt half and slightly beyond. Although the lines are shown to be continuous, it is to be understood that they may instead comprise intermittent lines of attachment. Preferably, the lines of attachment 54 are adhesive bond lines, though any attachment methods, such as thermal or ultrasonic bonding, may be employed.

The two spaced substantially parallel lines of attachment 54 at least partially delimit a non-attached longitudinally extending region 56 between the belt half 14 and the backsheet 22. This non-attached region 56 permits the elastic ribbons 48 or strands of the elasticised leg cuffs to snap back during manufacture. Thus, free ends of the elastic ribbons 48 or strands reside in the non-attached longitudinally extending region 56. To firmly anchor the elastic ribbons 48 within the absorbent structure 16, the joint 50 between the belt half 14 and the rear end region 32 further comprises a region 58 of attachment between the backsheet 22 and the belt half. The region 58 of attachment extends between the two spaced substantially parallel lines of attachment 54 over the elastic ribbons 48. Advantageously, the region 58 of attachment extends in the longitudinal direction beyond the point at which the two spaced parallel lines of attachment 54 terminate. The joint 50 between the belt half 14 and the rear end region32 may further comprise at least one region of bonding between the belt half and the topsheet 20. Such region may comprise substantially the entire surface of the belt half which lies adjacent the topsheet 20. This region of bonding may be suitably achieved by spray bonding the topsheet to the belt half.

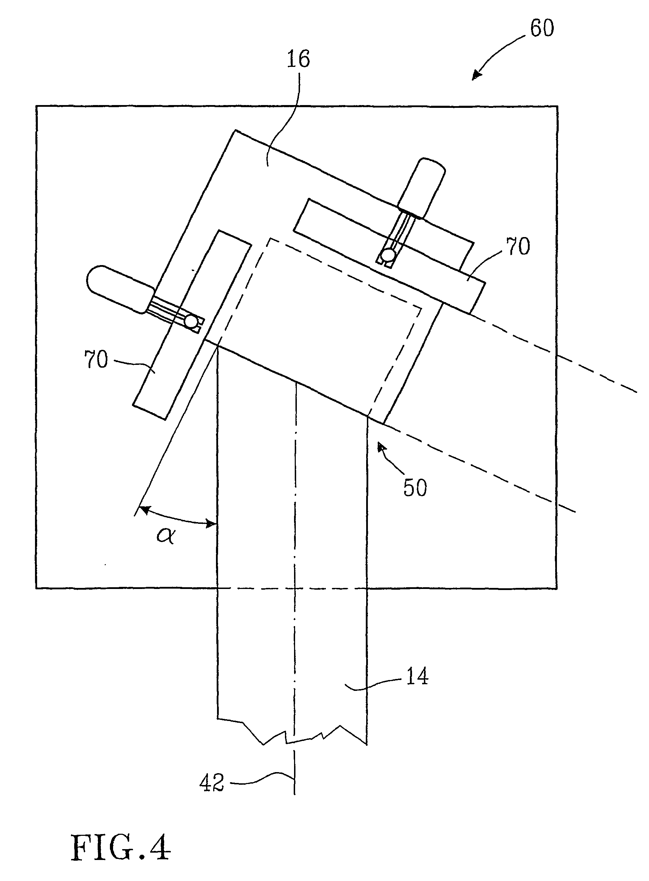

In accordance with the present invention, the joint 50 is designed to meet certain minimum requirements. The present inventors have discovered that, for the absorbent article to function satisfactorily, the joint 50 must be capable of withstanding a certain tension force applied to the belt halves at a certain angle α to the transverse axis of the absorbent structure for a certain minimum period of time. Thus, and in a manner which will be explained in greater detail below, the joint is subjected to a test procedure such that, and as illustrated in Fig. 4, a portion of the absorbent structure 16 is secured to a test rig, generally denoted reference numeral 60, and a load of 35 N is applied to the belt half 14 whilst the belt half external of the absorbent structure 16 is maintained at a predetermined angle α to the transverse axis of the absorbent structure. The time up to failure of the joint, i.e. when the belt half completely dissociates from the absorbent structure, is measured. The time to failure is hereinafter referred to as the release time of the joint. In accordance with the present invention, the minimum average release times of the joint should be:
when α = 10°, minimum t = 720 seconds;
when α = 20°, minimum t = 330 seconds;
when α = 25°, minimum t = 240 seconds;
when α = 30°, minimum t = 180 seconds; and
when α = 40°, minimum t = 75 seconds.

In a preferred embodiment of the invention, the following minimum average release times (t) of each belt half from the absorbent structure are attained:
when α = 10°, minimum t = 740 seconds;
when α = 20°, minimum t = 340 seconds;
when α = 25°, minimum t = 245 seconds;
when α = 30°, minimum t = 190 seconds; and
when α = 40°, minimum t = 80 seconds.

The minimum average release times are established in the following manner.

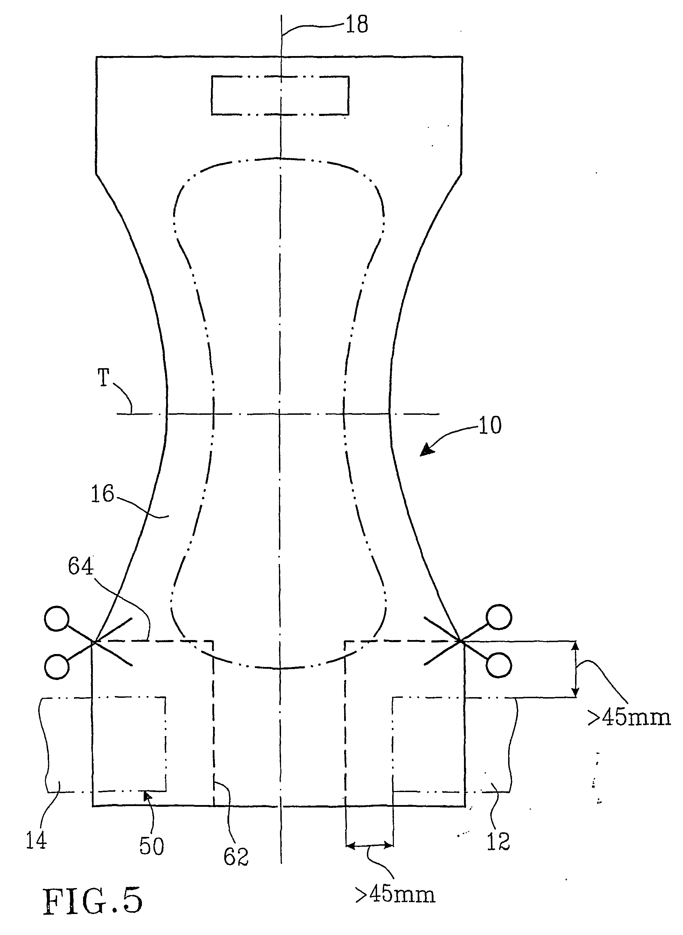

With reference to Fig. 5, a section of the rear panel 30 including the joint 50 is cut out from the absorbent structure 16. A first cut line 62 is made parallel to the longitudinal axis 18 of the absorbent article at least 45 mm from the end edge of the belt half 14 nearest the longitudinal axis 18. The first cut line 62 extends beyond the lower edge, i.e. that edge nearest the transverse axis T of the absorbent article, of the belt half 14 by at least 45 mm. The first cut line intersects a second cut line 64 extending parallel to the transverse axis T of the absorbent article at least 45 mm from the lower edge of the belt half 14. The thus cut out section of the absorbent structure 16 including the joint 50 is then clamped in the test rig 60 which will be described in greater detail in the following.

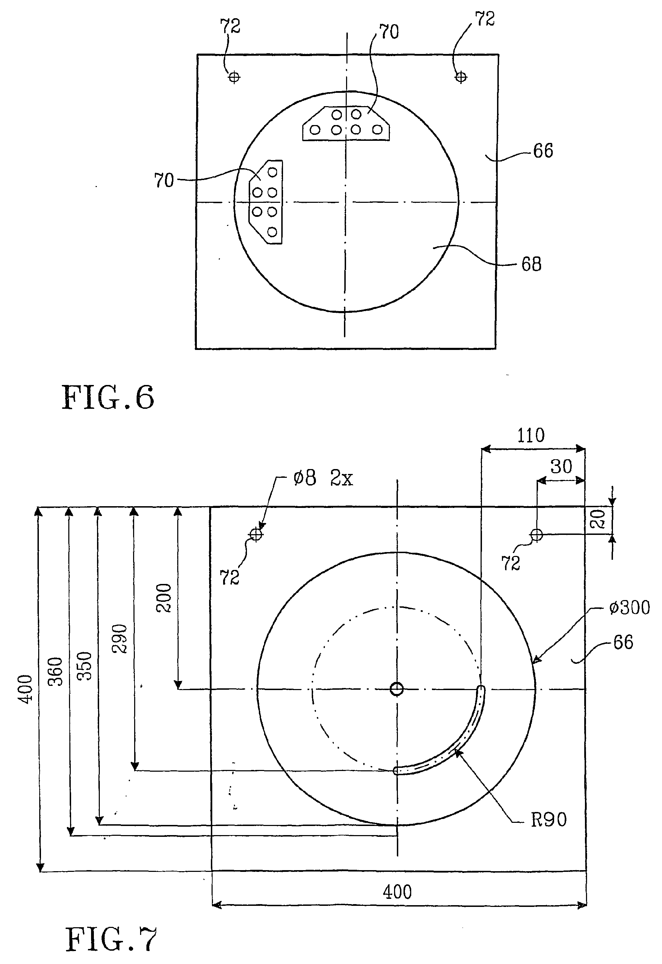

With reference to Fig. 6, the test rig 60 comprises a rectangular base plate 66 to which a rotatable plate 68 is mounted. A pair of clamps 70 spaced at 90° to each other are mounted on the rotatable plate 68. With reference to Fig. 4, the rotatable plate 68 can be rotated with respect to the rectangular base plate 66 such that suitable values of the angle α are obtainable. The test rig 60 is provided with locking means to enable the rotatable plate to be locked at angular positions at which desired values of α are obtained. The rectangular base plate 66 may be provided with holes 72 to enable the base plate to be maintained in a vertical position on a frame (not shown) or the like.

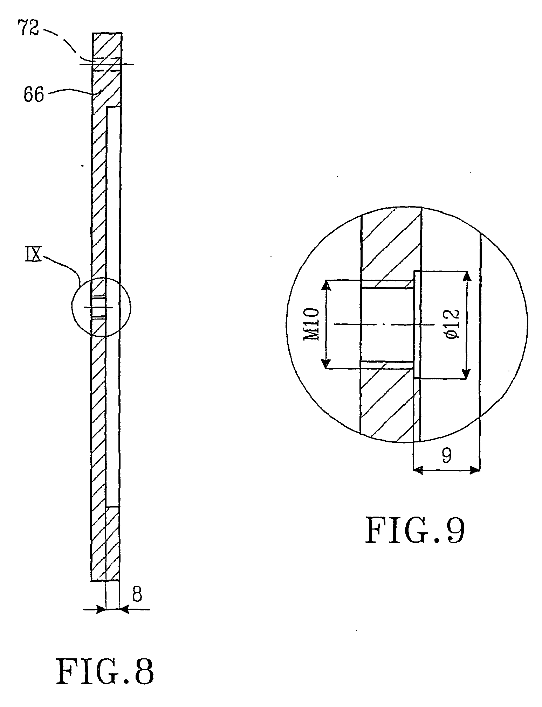

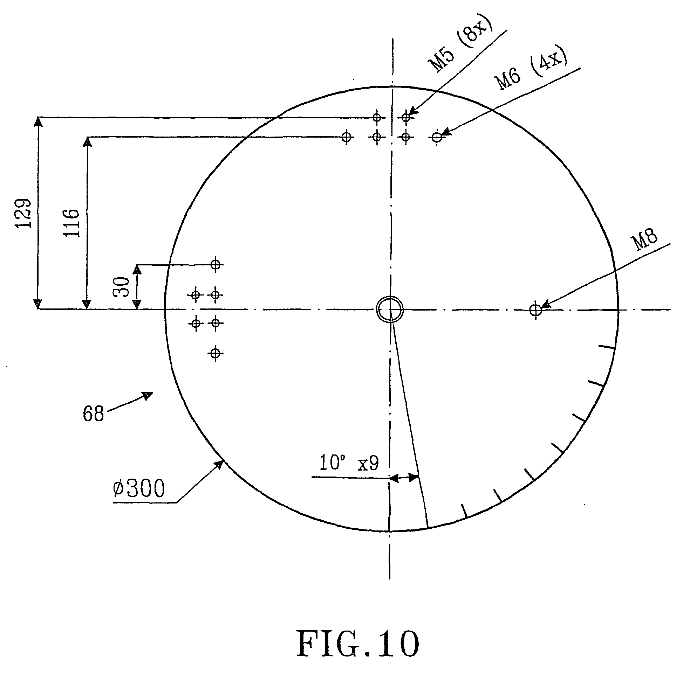

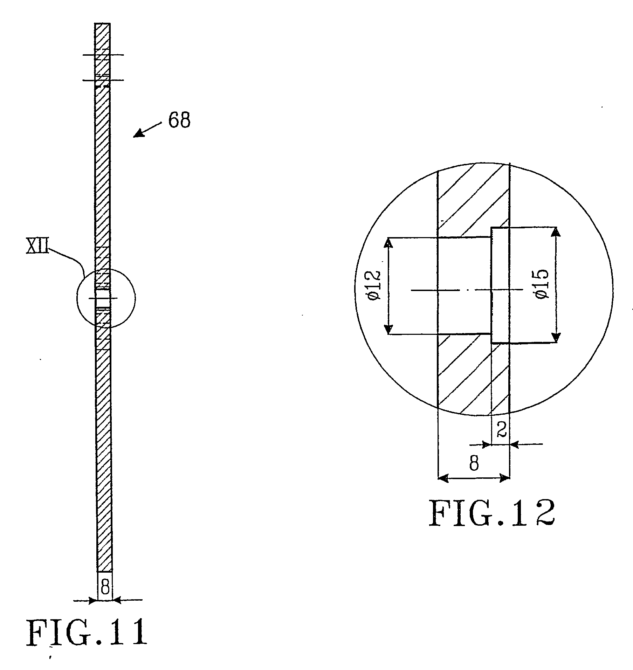

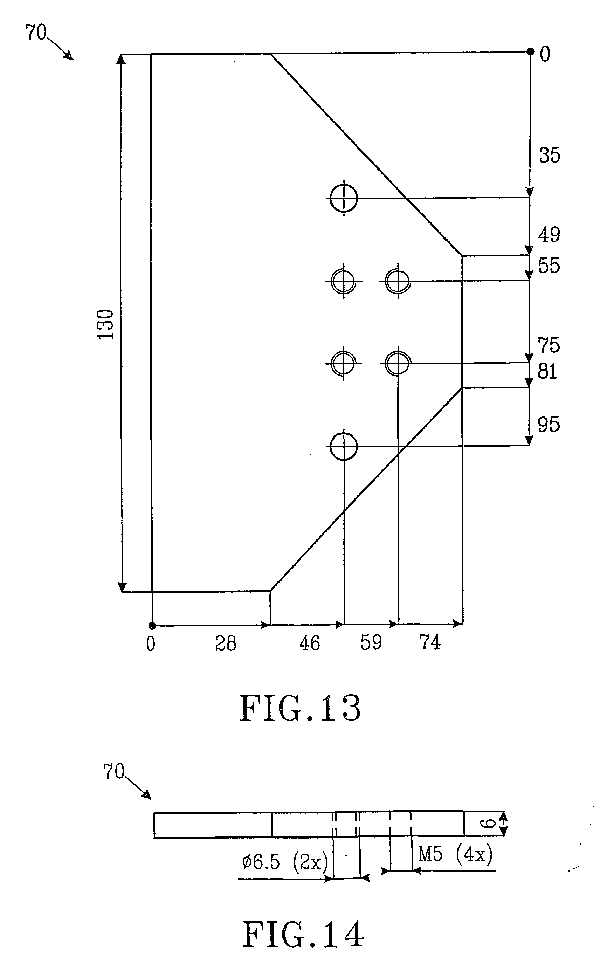

Exact dimensions and constructional details of the test rig 60 will be apparent from the attached Figs. 7 to 14.

The method for establishing the minimum average release times is the following.

Sections are cut out from fifty identical absorbent articles. In order to avoid the influence of aging of the articles on the test results, the articles should be no more than 6 months old, i.e. the test is to be performed on articles which have been manufactured during the past six months. The cut out section of a first absorbent structure 16 is secured between the clamps 70 of the test rig 60 as shown in Fig. 4. The orientation of the cut out section must be such that the clamps 70 clamp the cut out section along lines parallel to the transverse axis T and the longitudinal axis 18 of the absorbent article. In Fig. 4, the edges of the belt half 14 within the absorbent structure 16 are parallel to the transverse and longitudinal axes respectively. Thus, these edges are parallel to, and spaced from the clamps 70. In order to ensure that the clamps do not contact the belt half 14, a spacing of about 1 cm may be employed. The rectangular base plate 66 is held vertically and the rotatable plate 68 is rotated until an angle α of 10° is attained. The rotatable plate is locked at this position and a weight is clamped to the free end of the belt half 14. The weight is slowly released until it applies a tension to the belt half. The weight is then allowed to hang freely. The weight and clamp together apply a force of 35 N to the belt half. As soon as the weight is allowed to hang freely, a stop watch is started. As soon as the belt half completely dissociates from the absorbent structure, i.e. when the weight hits the floor, the stop watch is stopped and the elapsed time is noted.

The above procedure is repeated for nine further cut out sections at α = 10°.

For the next ten cut out sections, the above procedure is repeated for α = 20°.

Batches of ten cut out sections are then subjected to the above procedure, but for α = 25°, 30° and 40°.

### EXAMPLE

The above procedure was conducted on a belted absorbent article to ensure that its j oint 50 between each belt half and the absorbent structure is sufficiently strong. The following results were obtained (note that the elapsed time is given in minutes and seconds):

| **α =** | **10°** | **20°** | **25°** | **30°** | **40°** |
|---|---|---|---|---|---|
| Sample 1 | 12:31 | 05:35 | 03:21 | 04:23 | 01:49 |
| 2 | 10:16 | 05:42 | 05:51 | 04:14 | 01:52 |
| 3 | 12:52 | 06:27 | 04:41 | 03:25 | 01:25 |
| 4 | 13:34 | 07:10 | 03:37 | 02:58 | 01:17 |
| 5 | 11:05 | 05:43 | 04:28 | 02:59 | 01:45 |
| 6 | 11:45 | 05:21 | 04:01 | 03:29 | 00:52 |
| 7 | 16:24 | 07:11 | 03:59 | 02:18 | 01:25 |
| 8 | 12:07 | 05:00 | 04:39 | 02:39 | 01:09 |
| 9 | 11:27 | 04:57 | 03:27 | 03:36 | 00:46 |
| 10 | 11:34 | 04:15 | 03:28 | 03:36 | 01:19 |
| **Average:** | **12:21** | **05:44** | **04:09** | **03:21** | **01:21** |
| St.dev. | 01:41 | 00:57 | 00:46 | 00:39 | 00:22 |
| Min | 10:16 | 04:15 | 03:21 | 02:18 | 00:46 |
| Max | 16:24 | 07:11 | 05:51 | 04:23 | 01:52 |

Thus, the average release times for the joint of the EXAMPLE are:
when α = 10°, t = 741 seconds;
when α = 20°, t = 344 seconds;
when α = 25°, t = 249 seconds;
when α = 30°, t = 201 seconds; and
when α = 40°, t = 81 seconds.

The above EXAMPLE relates to a belted absorbent article in which the belt halves are substantially rectilinear, i.e. the upper and lower longitudinal edges of the belt halves are substantially parallel to the second longitudinal axis 42. Thus, the angle α may be determined either by measuring the angle as shown in Fig. 4, or by measuring the angle subtended by the second longitudinal axis 42. In cases in which the belt halves are non-rectilinear, i.e. the belt halves are curved, the angle α is determined by hanging the load of 35 N from the remote end of the belt half such that the load acts along the longitudinal axis of the belt half and determining the mid-point of the belt half between its upper and lower longitudinal edges at the joint 50. A vertical line will thus be attained between the mid point and the load. The angle that this vertical line subtends to the transverse axis T of the absorbent article corresponds to the angle α.

The invention is not restricted to the embodiments described above and shown in the drawings, but may be varied within the scope of the appended claims. For example, the topsheet 20 of the absorbent structure 16 may be provided with so-called standing gathers to assist in retaining bodily wastes within the confines of the absorbent structure.

## Claims

1. A belted absorbent article (10) comprising:
an absorbent structure (16) extending about a first longitudinal axis (18), said absorbent structure including a topsheet (20), a backsheet (22) and an absorbent batt (24) disposed between said topsheet and said backsheet, said absorbent structure having a transverse axis (T) dividing the absorbent structure into a front panel (26) terminating in a front end region (28) and a rear panel (30) terminating in a rear end region (32), said absorbent structure being delimited by opposed longitudinal edges (34) and opposed transverse edges (36), and
a pair of opposed belt halves (12, 14) attached to said absorbent structure (16) at said rear end region (32) of said rear panel (30) by a respective joint (50), each belt half extending about a second longitudinal axis (42) such that each belt half extends outwardly from a respective longitudinal edge (34) of the absorbent structure,
**characterized in that**
said joint (50) between each said belt half (12, 14) and said absorbent structure (16) is designed such that when each said belt half is subjected to a tension force of 35 N acting along said second longitudinal axis (42) and said second longitudinal axis creates an angle (α) to said transverse axis (T) of said absorbent structure, the following minimum average release times (t) of each belt half from said absorbent structure are attained:
when α = 10°, minimum t = 720 seconds;
when α = 20°, minimum t = 330 seconds;
when α = 25°, minimum t = 240 seconds;
when α = 30°, minimum t = 180 seconds; and
when α = 40°, minimum t = 75 seconds.

2. The article as claimed in claim 1, **characterized in that** the following minimum average release times (t) of each said belt half from said absorbent structure are attained:
when α = 10°, mininum t = 740 seconds;
when α = 20°, mininum t = 340 seconds;
when α = 25°, mininum t = 245 seconds;
when α = 30°, mininum t = 190 seconds; and
when α = 40°, mininum t = 80 seconds.

3. The article as claimed in any one of claims 1 and 2, **characterized in that** each said belt half (12, 14) is attached to said absorbent structure (16) at said rear end region (32) between said topsheet (20) and said backsheet (22).

4. The article as claimed in claim 3, **characterized in that** said joint (50) between each said belt half (12,14) and said rear end region (32) comprises two spaced substantially parallel lines of attachment (54) between each said belt half and said backsheet (22).

5. The article as claimed in claim 4, **characterized in that** said two spaced substantially parallel lines of attachment (54) are adhesive bond lines.

6. The article as claimed in claim 4 or 5, **characterized in that** said two spaced substantially parallel lines of attachment (54) at least partially delimit a non-attached longitudinally extending region (56) between each said belt half (12,14) and said backsheet (22) within which region ends of elastic ribbons (48) forming elasticised leg cuffs reside.

7. The article as claimed in claim 6, **characterized in that** said joint (50) between each said belt half (12, 14) and said rear end region (32) further comprises a region of attachment (58) between said backsheet (22) and each said belt half, said region of attachment extending between said two spaced substantially parallel lines of attachment (54) over said elastic ribbons (48).

8. The article as claimed in any one of claims 3 to 7, **characterised in that** sad joint (50) between each said belt half and said rear end region further comprises at least one region of bonding between each said belt half and said topsheet (20).

## Patentansprüche

1. Absorbierender Gegenstand (10) mit Gürtel umfassend:
eine absorbierende Struktur (16), die sich entlang einer ersten Längsachse (18) erstreckt, eine Oberlage (20), eine Decklage (22) und eine zwischen der Oberlage und der Decklage angeordnete Matte (24) umfasst sowie eine Querachse (T) aufweist, die die absorbierende Struktur in ein vorderes Feld (26), das in einem vorderen Endbereich (28) endet und ein hinteres Feld (30), das in einem hinteren Endbereich (32) endet, unterteilt, wobei die absorbierende Struktur durch gegenüberliegende Längskanten (34) und gegenüberliegende Querkanten (36) begrenzt ist, und
ein Paar gegenüberliegender Gürtelhälften (12, 14), die durch eine entsprechende Verbindung (50) im hinteren Endbereich (32) des hinteren Feldes (30) an der absorbierenden Struktur (16) angebracht sind, wobei sich jede Gürtelhälfte entlang einer zweiten Längsachse (42) erstreckt, so dass sich jede Gürtelhälfte von einer entsprechenden Längskante (34) der absorbierenden Struktur nach außen erstreckt,
**dadurch gekennzeichnet, dass**
die Verbindung (50) zwischen der jeweiligen Gürtelhälfte (12, 14) und der absorbierenden Struktur (16) derart ausgestaltet ist, dass die folgenden jeweiligen minimalen durchschnittlichen Lösezeiten (t) der Gürtelhälften von der absorbierenden Struktur erzielt werden, wenn die Gürtelhälften jeweils einer Zugkraft von 35 N, die entlang der zweiten Längsachse (42) wirkt, ausgesetzt werden und die zweiten Längsachsen einen Winkel α zur Querachse (T) der absorbierenden Struktur erzeugen:
wenn α = 10°, minimal t = 720 s;
wenn α = 20°, minimal t = 330 s;
wenn α = 25°, minimal t = 240 s;
wenn α = 30°, minimal t = 180 s; und
wenn α = 40°, minimal t = 75 s.

2. Gegenstand nach Anspruch 1, **dadurch gekennzeichnet, dass** die folgenden jeweiligen minimalen durchschnittlichen Lösezeiten (t) der Gürtelhälften von der absorbierenden Struktur erzielt werden:
wenn α = 10°, minimal t = 720 s;
wenn α = 20°, minimal t = 340 s;
wenn α = 25°, minimal t = 245 s;
wenn α = 30°, minimal t = 190 s; und
wenn α = 40°, minimal t = 80 s.

3. Gegenstand nach einem der Ansprüche 1 und 2, **dadurch gekennzeichnet, dass** jede Gürtelhälfte (12, 14) zwischen der Oberlage (20) und der Decklage (22) im hinteren Endbereich (32) an der absorbierenden Struktur (16) angebracht ist.

4. Gegenstand nach Anspruch 3, **dadurch gekennzeichnet, dass** die Verbindung (50) zwischen jeder Gürtelhälfte (12, 14) und dem hinteren Endbereich (32) zwei beabstandete im Wesentlichen parallele Anbringungslinien (54) zwischen jeder Gürtelhälfte und der Decklage (22) umfasst.

5. Gegenstand nach Anspruch 4, **dadurch gekennzeichnet, dass** die zwei beabstandeten im Wesentlichen parallelen Anbringungslinien (54) Klebemittelverbindungslinien sind.

6. Gegenstand nach Anspruch 4 oder 5, **dadurch gekennzeichnet, dass** die zwei beabstandeten im Wesentlichen parallelen Anbringungslinien (54) wenigstens teilweise einen nicht angebrachten längslaufenden Bereich (56) zwischen jeder Gürtelhälfte (12, 14) und der Decklage (22) begrenzen in welchem Bereich Enden elastischer Fäden (48) elastische Beinbündchen bilden.

7. Gegenstand nach Anspruch 6, **dadurch gekennzeichnet, dass** die Verbindung (50) zwischen jeder Gürtelhälfte (12, 14) und dem hinteren Endbereich (32) ferner einen Anbringungsbereich (58) zwischen der Decklage (22) und jeder Gürtelhälfte umfasst, wobei sich der Anbringungsbereich zwischen zwei beabstandeten im Wesentlichen parallelen Anbringungslinien (54) über die elastischen Fäden (48) erstreckt.

8. Gegenstand nach einem der Ansprüche 3-7, **dadurch gekennzeichnet, dass** die Verbindung (50) zwischen jeder Gürtelhälfte und dem hinteren Endbereich ferner wenigstens einen Verbindungsbereich zwischen jeder Gürtelhälfte und der Oberlage (20) umfasst.

## Revendications

1. Article absorbant à ceinture (10), comprenant :
une structure absorbante (16) s'étendant autour d'un premier axe longitudinal (18), ladite structure absorbante comprenant une feuille supérieure (20), une feuille de revers (22) et un nappage absorbant (24) disposé entre ladite feuille supérieure et ladite feuille de revers, ladite structure absorbante comportant un axe transversal (T) divisant la structure absorbante en un panneau avant (26) s'achevant dans une région d'extrémité avant (28) et un panneau arrière (30) s'achevant dans une région d'extrémité arrière (32), ladite structure absorbante étant délimitée dans des bords longitudinaux opposés (34) et des bords transversaux opposés (36), et
une paire de moitiés de ceinture opposées (12, 14) fixées à ladite structure absorbante (16) dans ladite région d'extrémité arrière (32) dudit panneau arrière (30) par un raccord respectif (50), chaque moitié de ceinture s'étendant autour d'un deuxième axe longitudinal (42) de telle sorte que chaque moitié de ceinture s'étende vers l'extérieur à partir d'un bord longitudinal respectif (34) de la structure absorbante,
**caractérisé en ce que**:
ledit raccord (50) entre chacune desdites moitiés de ceinture (12, 14) et ladite structure absorbante (16) est conçu de telle sorte que, lorsque chacune desdites moitiés de ceinture est soumise à une force de traction de 35 N agissant le long dudit deuxième axe longitudinal (42) et que ledit deuxième axe longitudinal crée un angle (α) par rapport audit axe transversal (T) de ladite structure absorbante, les temps de relâchement moyens minimum (t) suivants de chaque moitié de ceinture par rapport à ladite structure absorbante sont atteints:
lorsque α = 10°, t minimum = 720 secondes;
lorsque α = 20°, t minimum = 330 secondes ;
lorsque α = 25°, t minimum = 240 secondes ;
lorsque α = 30°, t minimum = 180 secondes ; et
lorsque α = 40°, t minimum = 75 secondes.

2. Article selon la revendication 1, **caractérisé en ce que** les temps de relâchement moyens minimum (t) suivants de chacune desdites moitiés de ceinture par rapport à ladite structure absorbante sont atteints :
lorsque α = 10°, t minimum = 740 secondes ;
lorsque α = 20°, t minimum = 340 secondes ;
lorsque α = 25°, t minimum = 245 secondes ;
lorsque α = 30°, t minimum = 190 secondes ; et
lorsque α = 40°, t minimum = 80 secondes.

3. Article selon l'une quelconque des revendications 1 et 2, **caractérisé en ce que** chacune desdites moitiés de ceinture (12, 14) est fixée à ladite structure absorbante (16) dans ladite région d'extrémité arrière (32) entre ladite feuille supérieure (20) et ladite feuille de revers (22).

4. Article selon la revendication 3, **caractérisé en ce que** ledit raccord (50) entre chacune desdites moitiés de ceinture (12, 14) et ladite région d'extrémité arrière (32) comprend deux lignes de fixation espacées sensiblement parallèles (54) entre chacune desdites moitiés de ceinture et ladite feuille de revers (22).

5. Article selon la revendication 4, **caractérisé en ce que** lesdites deux lignes de fixation espacées sensiblement parallèles (54) sont des lignes de fixation adhésives.

6. Article selon les revendications 4 ou 5, **caractérisé en ce que** lesdites deux lignes de fixation espacées sensiblement parallèles (54) délimitent au moins partiellement une région non-fixée s'étendant longitudinalement (56) entre chacune desdites moitiés de ceinture (12, 14) et ladite feuille de revers (22), région à l'intérieur de laquelle se trouvent des extrémités de rubans élastiques (48) constituant des revers de patte élastiques.

7. Article selon la revendication 6, **caractérisé en ce que** ledit raccord (50) entre chacune desdites moitiés de ceinture (12, 14) et ladite région d'extrémité arrière (32) comprend de plus une région de fixation (58) entre ladite feuille de revers (22) et chacune desdites moitiés de ceinture, ladite région de fixation s'étendant entre lesdites deux lignes de fixation espacées sensiblement parallèles (54) sur lesdits rubans élastiques (48).

8. Article selon l'une quelconque des revendications 3 à 7, **caractérisé en ce que** ledit raccord (50) entre chacune desdites moitiés de ceinture et ladite région d'extrémité arrière comprend de plus au moins une région de fixation entre chacune desdites moitiés de ceinture et ladite feuille supérieure (20).
